# EUROPEAN PATENT APPLICATION

(11) **EP 1 623 741 A2**
(43) Date of publication of application: **08.02.2006**
(21) Application number: 05254582.9
(22) Date of filing: 22.07.2005
(51) Int. Cl.: A61P 17/14, A61K 31/415, C07D 231/10

(54) **Cannabinoid receptor ligands for hair growth modulation**

(30) Priority: 22.07.2004 IN MU07782004; 20.12.2004 IN MU13682004
(71) Applicant: CADILA HEALTHCARE LTD., Ahmedebad 380 015, Gujarat (IN)
(72) Inventor: Lohray, Braj Bhushan, c/o Cadila Healthcare Ltd., Ahmedabad 380015 (IN); Lohray, Vidya Bhushan, c/o Cadila Healthcare Ltd., Ahmedabad 380015 (IN); Jain, Mukui K, c/o Cadila Healthcare Ltd., Ahmedabad 380015 (IN); Srivastava, Brigesh K., c/o Cadila Healthcare Ltd., Ahmedabad 380015 (IN)
(74) Representative: Hale, Stephen Geoffrey

(57) **Abstract**

The growth of body and/or head/cranial hair on mammalian organisms, for example humans, is modulated by administering thereto, whether topically and/ or systemically, therapeutically effective amounts of at least one cannabinoid receptor ligand, with or without other suitable therapeutically active agents.

## Description

### FIELD OF INVENTION

The present invention discloses a process for modulating the growth of body and/or head/cranial hair on mammalian organisms, for example humans, by administering thereto, whether topically and/or systemically, therapeutically effective amounts of at least one cannabinoid modulators, in combination with or without other suitable therapeutically active agents.

### BACKGROUND AND PRIOR ART

Cannabinoids are present in Indian hemp *Cannabis sativa* and have been well known for their medicinal properties for ages. Cannabinoids as a therapeutic agents is however a recent phenomenon. (Williamson E. M. & Evans E. J. *Drugs* 2000 Dec; 60(6): 1303-14) Research in this area over the last decade have provided very important information on the cannabinoid receptors and their agonists and antagonists.

There has been an increased interest among the different pharmaceutical companies in developing drugs for the treatment of diseases connected with disorders of the cannabinoid systems (Greenberg D. A., *Drugs News & Perspectives* 1999; 12: 458; Kulkarni S. K. & Ninan, *Indian Journal of Pharmacology* 2001; 33: 170-184; Piomelli D et. al., *Trends Pharmacol Sci.* 2000 Jun; 21(6): 218-24). Several compounds which are either CB₁, CB₂ &/or CB₃ antagonists have been reported and are under various stages of development for e.g. SR-141716 A (Sanofi), CP-272871 (Pfizer), LY-320135 (Eli Lily), AM-630 (Alexis), SR-144528 (Sanofi) etc.

Compounds which mimic the actions of the cannabinoids are useful for preventing or reversing the symptoms that can be treated with cannabis, some of its derivatives, and synthetic cannabinoids in a human or other mammalian subject. Thus compounds which are modulators of cannabinoid receptors are known to be useful in the treatment or amelioration of disorders, in mammals, such as (a) pulmonary disorders including asthma, chronic bronchitis; (b) ocular disorders such as glaucoma; (c) allergies and allergic reactions; (d) inflammatory conditions like arthritis, inflammatory bowel disease; (e) pain; (f) immune system disorders like AIDS, lupus; (g) allograft rejections; (h) central nervous system disorders like Torette's syndrome, Parkinson's disease, Huntington's disease, epilepsy, various psychotic disorders like depression, manic depression etc.; (i) vomiting, nausea and vertigo; (j) obesity; (k) cognitive disorders such as Alzheimer's disease; (1) schizophrenia; (m) smoking cessation.

Use of cyclooxygenase or a lipoxygenase inhibitor as hair growth modulators have been described in US 6465421 & US 5928654.

We herein disclose compounds, which are cannabinoid receptors ligands, as hair growth modulators suitable for use in mammals either alone or in combination with other suitable therapeutically active agents.

### DESCRIPTION OF THE ACCOMPANYING DRAWINGS:

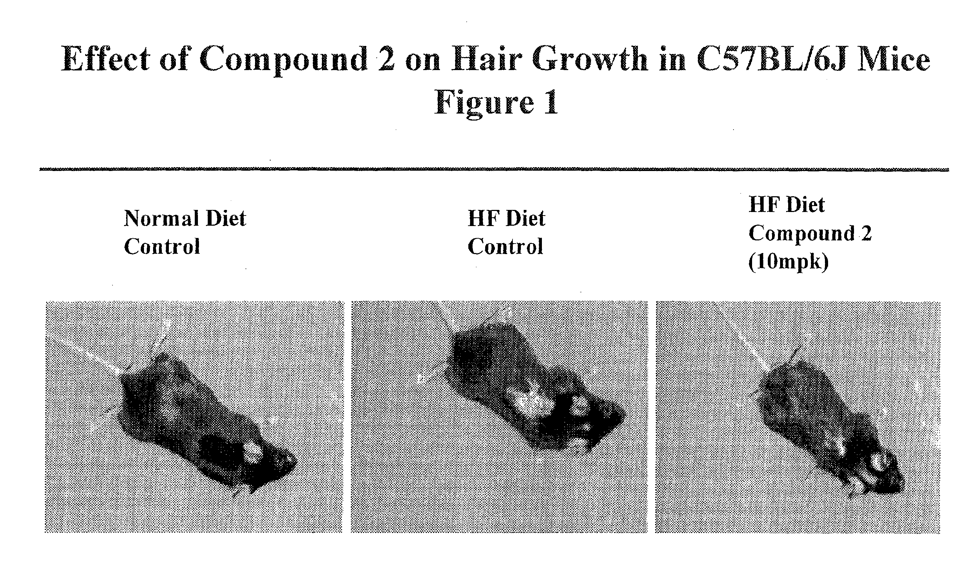

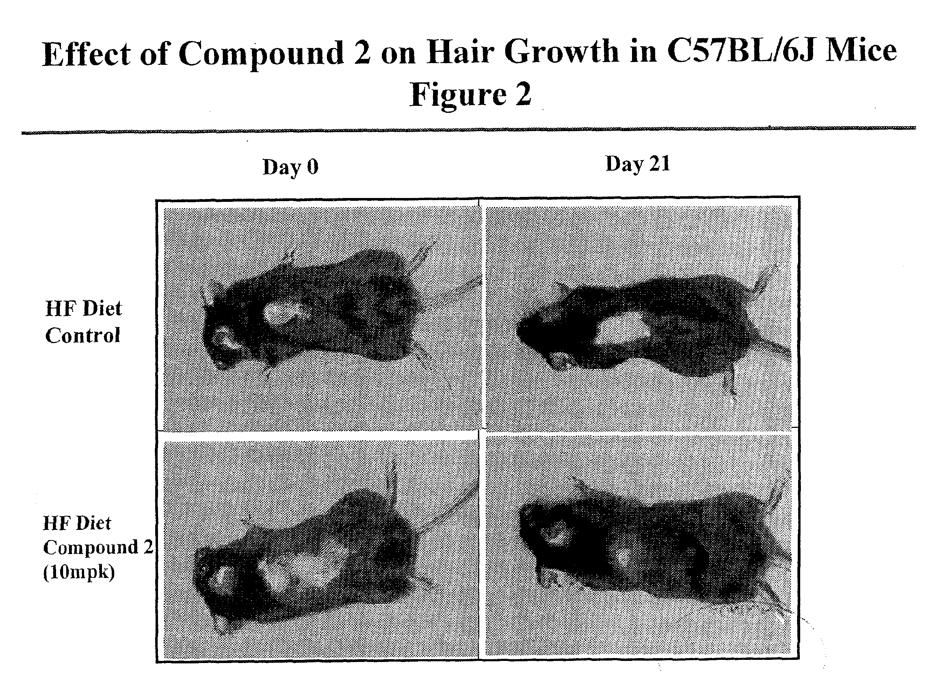

Figure 1 and 2: Effect of Compound 2 on Hair Growth in C57BL/6J Mice

### DETAILED DESCRIPTION

The present invention discloses compounds, which are cannabinoid receptors ligands, suitable for modulating hair growth in mammals. Several compounds which act as cannabinoid receptors ligands, their preparation and their use in medicine have been reported in US 20050101592, US 20050096379, US 5925768, US 6344474, US 6028084, US 5462960, EP 0656354, US 6432984, US 6509367, US 5624941, US 20010053788, US 6476060, US 2004039024, EP1230222, EP 122952, FR 2816938, FR 2761266, FR 2800375, EP 0656354, EP 0576357, WO 0170700, WO 02076949, WO 2005044822, WO 2004096801, WO 2004094429, WO 2004096794, WO 2004094421, WO 2004094417, WO 2004096763, WO 200435566, WO 2004048317, WO 2004037823, WO 2004017920, WO 2004029204, WO 2004026301, WO 2004021974, WO 03082833, WO 03027076, WO 03026648, WO 03026647, WO 03020217, WO 03082191, WO 03084930, WO 03084943, WO 0228346, WO 0158450, WO 0185092, WO 0132663, WO 0132629, WO 9719063. Other compounds having similar activity have been disclosed in *J Pharmacology & Experimental Therapeutics,* **2003,** 306(1), 363-370; *Bioorganic Medicinal Chemistry,* **1997,** 5, 1591-1600; *J Med. Chem.* **1999,** 42, 769-776; *Bioorg. Med. Chem. Lett.,* **1999,** 9, 2233-2236; *Bioorg. Med Chem.* **2003,** 11, 251-263; *Bioorg. Med. Chem.* **2003,** 11, 3121-3132; *Bioorg. Med. Chem.* **2004,** 12, 393-404, *J Biological Chemistry,* **1996,** 271, 6941-6946; *J Med. Chem.,* **2002,** 45, 1748-1756; *J Med. Chem.,* **2002,** *45, 2708-2719; J Med. Chem.,* **2002,** *45, 3649-3659; J Med. Chem.,* **2002,** *45, 1447-1459; J Med. Chem.,* **2003,** *46, 642-645; J Med Chem.,* **2004,** 47, 627-643; *J Pharmacology & Experimental Therapeutics,* **2002,** 301(3), 963-968; *Molecular Pharmacology,* **2002,** 62(6), 1274-1287; *Drugs Fut.,* **2002,** 27 (Suppl. A): *XVIIth Int. Symposium on Medicinal Chemistry, Chem. Pharm. Bull.,* **2002**, 50, 1109-1113. However, use of such compounds as modulators of hair growth has not been envisaged.

In a specific embodiment, the present invention discloses use of compounds disclosed in any of the above references or pharmaceutical compositions containing them as modulators of hair growth in mammals either alone or in combination with one or more other suitable therapeutic agents.

A "cannabinoid receptor ligand" according to the present invention includes a cannabinoid receptor antagonist, agonist or an inverse agonist.

A "hair growth modulator" according to the present invention includes a compound which hair growth stimulant or a repressant.

In a further embodiment the present invention provides method of modulating hair growth in mammals by treatment with compounds which are ligands of the cannabinoid receptors.

In one of its embodiments the suitable cannabinoid receptor ligands for use according to the present invention bind to CB₁, CB₂ and / or CB₃ receptors.

In a preferred embodiment, the compounds may be selected from the group which are preferentially antagonist or an inverse agonists of the CB₁ receptor.

Non-limiting examples of cannabinoid receptor ligands are Rimonabant or its analogues, SLV-319 and the like.

In a further embodiment, the present invention discloses compound of formula (I) or pharmaceutical composition containing the same, as modulators of hair growth in mammals, 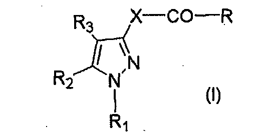 wherein,
R₁ represents substituted or unsubstituted groups selected from (C₃-C₇)cycloalkyl, (C₃₋C₇)cycloalkenyl, bicycloalkyl, bicycloalkenyl, aryl, aralkyl, heterocyclyl, heteroaryl, heterocyclyl(C₁-C₁₂)alkyl & heteroar(C₁-C₁₂)alkyl;
R₂ represents a substituted or unsubstituted single or fused heteroaromatic or a heterocyclic group containing one or more heteroatoms selected from N, O or S;
R₃ represents hydrogen, halo, cyano, nitro, (C₁-C₁₂) substituted or unsubstituted alkyl, (C₁-C₁₂) substituted or unsubstituted haloalkyl, hydroxyalkyl, cycloalkyl, alkylsulfonyl groups;
X is either a direct bond or a group -(CH₂)ₙN(R₄)-, wherein R₄ is H, or a (C₁-C₃)alkyl and n is 0-2;
R represents -NR₅R₆ where R₅ is either H or (C₁-C₆) alkyl; R₆ is 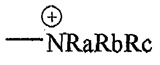 or -NR_{b}R_{c} where Rₐ is (C₁-C₆)alkyl or Rₐ forms a bridge with one of the atoms of the heterocyclic radical formed by -NR_{b}Rc;
R_{b} and R_{c} represents substituted or unsubstituted groups selected from alkyl, aralkyl or alkenyl or R_{b} & R_{c} together with the nitrogen atom to which they are bonded, form a 5 to 8 membered saturated or unsaturated heterocyclic radical which may be optionally substituted and may be fused.

In yet another embodiment, the present invention discloses compound of formula (Ia) or pharmaceutical composition containing the same, as regulators/promoters/stimulators of hair growth in mammals, 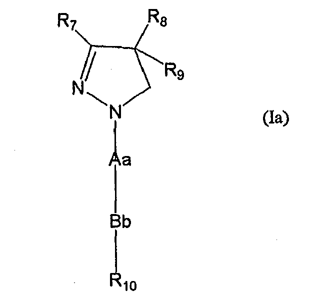 wherein,
'R₇' and 'R₈' are the same or different and represent phenyl, thienyl or pyridyl groups, which may be optionally substituted with 1-3 substituents Y, which may be same or different and selected from the group C₁₋₃-alkyl or alkoxy, hydroxy, halogen, trifluoromethyl, trifluromethylthio, trifluromethoxy, nitro, amino, mono- or dialkyl (C₁₋₂)-amino, mono- or dialkyl (C₁₋₃)-alkyl sulfonyl, dimethylsulfamido, C₁₋₃₋alkoxycarbonyl, carboxyl, trifluromethylsulfonyl, cyano, carbamoyl and acetyl, or R₇ and/or R₈ represent naphtyl; 'R₉' represents hydrogen, hydroxy, C₁₋₃-alkoxy, acetyloxy or propionyloxy;
- 'Aa' represents one of the groups (i), (ii), (iii), (iv) or (v) 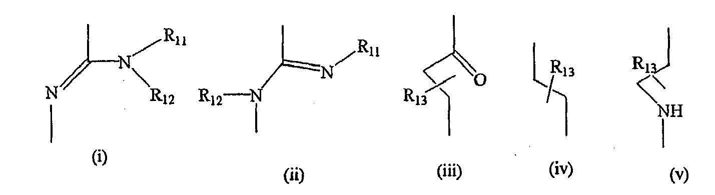 wherein
- 'R₁₁' and 'R₁₂' independently of each other represent hydrogen or C₁₋₈ branched or unbranched alkyl or C₃₋₈ cycloalkyl or 'R₁₁' represents acetamido or dimethylamino or 2,2,2-trifluroethyl or phenyl or pyridyl with the proviso that R₁₂ represents hydrogen
- 'R₁₃' represents hydrogen or C₁₋₃ unbranched alkyl;
- 'Bb' represents sulfonyl or carbonyl;
- 'R₁₀' represents benzyl, phenyl, thienyl or pyridyl which may be substituted with 1,2 or 3 substitutents Y, which can be the same or different, or 'R₁₀' represents C₁₋₈ branched or unbranched alkyl or C₃₋₆ cycloalkyl, or 'R₁₀' represents naphthyl.

The present invention also envisages the use of compounds, which are potentially suitable as cannabinoid receptors ligands as modulators of hair growth in mammals.

The present invention also discloses use of cannabinoid receptors ligands, in combination with other suitable therapeutically active agents for e.g. an inhibitor of cyclooxygenase or 5-lipoxygenase, or other hair growth modulators as are known in the art, as modulators of hair growth in mammals. Preferably, the other therapeutically active agent may be selected from Dutasteride, Finasteride, Minoxidil, Fluorominoxidil, Fluridil, Viprostol, Trequinsin hydrochloride, Namindil and Procyanidin B-2.

The quantity of active component, according to the present invention, in the pharmaceutical composition and unit dosage form thereof may be varied or adjusted widely depending upon the particular application method, the potency of the particular compound and the desired concentration. Generally, the quantity of active component will range between 0.5 % to 90 % by weight of the composition.

The precise dose and method of administration of cannabinoid receptors ligands to be used according to the present invention, will be determined by a number of factors, which will be apparent to those skilled in the art, in light of the disclosure herein.

Any suitable cannabinoid receptors ligands may be employed. A cannabinoid receptors ligands will be suitable if:
(a) at the dose and method of administration to the mammalian subject, it is not acutely toxic, and does not result in chronic toxicity disproportionate to the therapeutic benefit derived from treatment; and
(b) at the dose and method of administration to the mammalian subject it modulates hair growth in the subject.

Methods for conducting toxicity studies are known in the art.

The patient is preferably mammalian. In one embodiment the patient in which hair growth is modulated is a human. In another embodiment, it is a domestic animal such as cat, dog or horse.

In one embodiment of the invention, there is provided a method of modulating hair growth in mammalian patient in need thereof. The method comprises: selecting a patient in need of modulating hair growth, and administering a suitable cannabinoid receptor ligand.

In an embodiment is provided a topical formulation comprising atleast one cannabinoid receptor ligand, as a hair growth stimulant. The topical formulation may optionally contain one or more further hair growth stimulant. The formulation may further comprise other pharmaceutically acceptable excipients, suitable for suitably formulating the composition. The formulation may be prepared by techniques known in the art.

In one embodiment of the invention, there is provided a kit containing a cannabinoid receptor ligand and a pharmaceutically acceptable excipient. In one embodiment, the kit further comprises of, instructions for administering the cannabinoid receptor ligand to modulate hair growth in a mammalian subject. In yet another embodiment the kit still further comprises a means to administer the cannabinoid receptor ligand. Such kits may be prepared by techniques known.

Representative compounds suitable for carrying out the present invention includes:

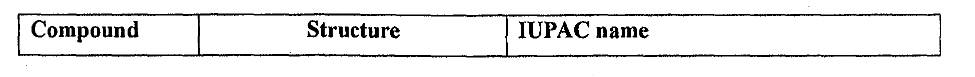

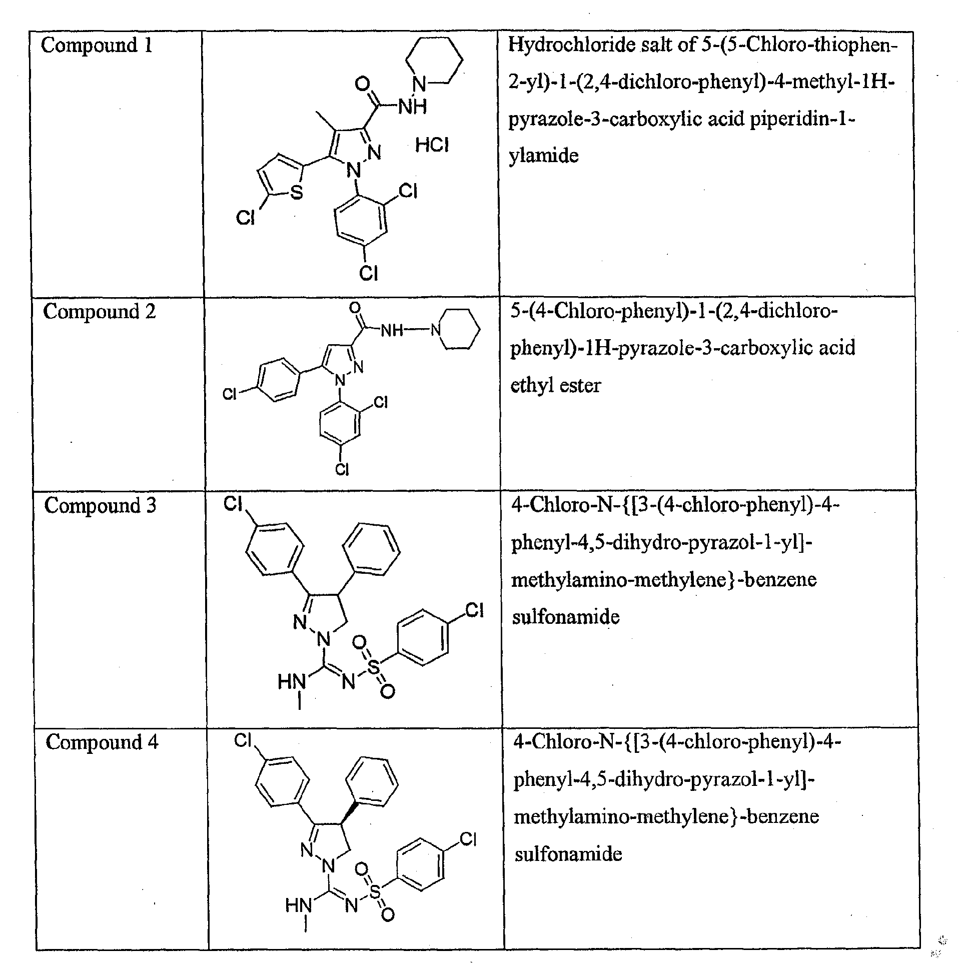

The present invention is illustrated by the following examples, which are provided for the sake of illustrations only and should not be construed as limiting the scope of the invention in any way.

### Example

### Effect of Compound 1 and Compound 2 on hair growth and body weight in male C57BL/6 mice

The C57BL/6 mice were housed on a lightdark cycle in a room with temperature (22 ±2°C) and humidity control. They were fed either a high-fat diet (HFD) (49% fat, 18% protein, 33% carbohydrate) or a standard mouse diet (STD) (8% fat, 19% protein, 73% carbohydrate). Six-week-old C57BL/6J male mice were given HFD or STD diets for 17 wks before drug treatment started. This diet treatment caused significantly higher body weight gain in high-fat fed male animals as compared to normal diet animals. Simultaneously, the animals in the high fat diet fed group showed significant loss of body hair, especially on the back. After this, mice were weighed and treated as per the following three groups, while the diet treatment continued.

High-fat diet fed & vehicle treated (HFD-V),

High-fat diet fed & treated with 10 mg/kg Compound 2 (HFD-R 10 mg)

High-fat diet fed & treated with 10 mg/kg Compound 1 (HFD-ZY 10 mg)

Compound 1 and Compound 2 were administered orally in distilled water with 0.1 % Tween 80 one hour before the onset of the dark phase.

The animals were observed daily. Body weights were recorded daily, and the animals were photographed on 28^{th} day on the back.

The results are mentioned in Table 1 and Table 2. It indicates that the treatment with 10mg/kg Compound 1, has significantly enhanced the growth of body hair. The same treatment has resulted decrease in body weight.

**Table 1: Effect of different treatments on the hair growth on male C57BL/6J mice Table indicate the bald area (where hair growth or pigmentation has not appeared) after 28^{th} day of dosing (n=6)**

| **Group** | **Area (cm**^{**2**}**) (Mean ± Standard Error Mean)** | | |
|---|---|---|---|
| HFD-V | 0.346 | ± | 0.079 |
| HFD-Compound 2 | 0.156 | ± | 0.068 |
| HFD- Compound 1 | 0.011 | ± | 0.223 |

**Table 2: Effect of differet treatments on the body weight in male C57BL/6J mice (n=6)**

| **Group** | **Body Weight on Day 0 (g)** | | |
|---|---|---|---|
| HFD-V | 30.5 | ± | 1.3 |
| HFD- Compound 2 | 30.5 | ± | 1.6 |
| HFD- Compound 1 | 29.2 | ± | 1.1 |

| Group | Body Weight on Day 28 (g) | | |
|---|---|---|---|
| HFD-V | 29.7 | ± | 1.1 |
| HFD- Compound 2 | 27.1 | ± | 1.1 |
| HFD- Compound 1 | 26.9 | ± | 1.1 |

| Group | Change in Body Weight (g) in 28 days versus Day 0 | | |
|---|---|---|---|
| HFD-V | -0.8 | ± | 0.4 |
| HFD- Compound 2 | -3.5 | ± | 0.8 |
| HFD- Compound 1 | -1.7 | ± | 0.5 |

| Group | Change in Body Weight (g) versus HFD-V | | |
|---|---|---|---|
| HFD-V | | | |
| HFD- Compound 2 | -9.7 | ± | 2.8 |
| HFD- Compound 1 | -3.3 | ± | 1.8 |

These data indicate that compound 1 and compound 2 are able to stimulate hair growth in addition to decreasing body weight.

In general throughout the text except where otherwise indicated any alkyl, alkenyl, (bi)cycloalkyl, (bi)cycloalkenyl, aryl, aralkyl, heteroaryl, heterocyclyl, haloalkyl, hydroxyalkyl or alkylsufonyl may for example have up to 12, more narrowly up to 6, carbon atoms in a carbon chain and up to 12, more narrowly up to 6, carbon atoms in a ring system, and the substitutuents in a substituted group may for example be the substituents Y.

The modulation, for example stimulation, of hair growth for cosmetic purposes in mammals, especially humans, is a very important activity, and the cannabinoid receptor ligands find use for such cosmetic purposes.

## Claims

1. Use of a cannabinoid receptor ligand for modulating hair growth in a mammalian subject.

2. Use according to claim 1, wherein the cannabinoid receptor ligand binds to a cannabinoid receptors subtype selected from CB₁, CB₂ and CB₃ receptors.

3. Use according to claim 1, wherein the cannabinoid receptor ligand is selected from at least one of the following,
(a) the cannabinoid receptor ligand of formula 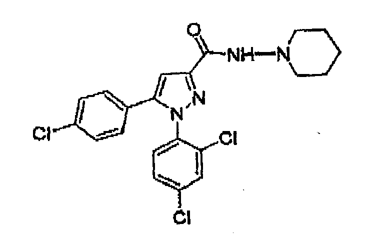
(b) cannabinoid receptor ligands of formula 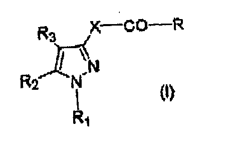
where
R₁ represents a substituted or unsubstituted group selected from (C₃₋C₇)cycloalkyl, (C₃-C₇)cycloalkenyl, bicycloalkyl, bicycloalkenyl, aryl, aralkyl, heterocyclyl, heteroaryl, heterocyclyl(C₁-C₁₂)alkyl & heteroar(C₁-C₁₂)alkyl;
R₂ represents a substituted or unsubstituted single or fused heteroaromatic or a heterocyclic group containing one or more heteroatoms selected from N, O or S;
R₃ represents hydrogen, or a halo, cyano, nitro, (C₁-C₁₂) substituted or unsubstituted alkyl, (C₁-C₁₂) substituted or unsubstituted haloalkyl, hydroxyalkyl, cycloalkyl or alkylsulfonyl group;
X is either a direct bond or a group -(CH₂) ₙN(R₄)-, where R₄ is H or (C₁₋C₃)alkyl and n is 0, 1 or 2;
R represents -NR₅R₆, where R₅ is either H or (C₁-C₆) alkyl; R₆ is - NRₐR_{b}R_{c} or -NR_{b}R_{c} where Rₐ is (C₁-C₆)alkyl or Rₐ forms a bridge with one of the atoms of the heterocyclic radical formed by -NR_{b}R_{c}; R_{b} and R_{c} separately represent substituted or unsubstituted groups selected from alkyl, aralkyl and alkenyl or R_{b} & R_{c} together with the nitrogen atom to which they are bonded form a 5 to 8 membered saturated or unsaturated heterocyclic radical which is optionally substituted and may be fused, and
(c) cannabinoid receptor ligands of formula 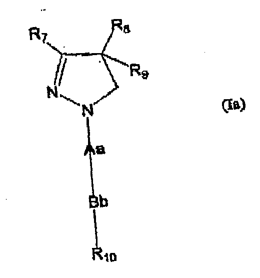 where
R₇ and R₈ are the same or different and represent phenyl, thienyl or pyridyl groups, which are optionally substituted with 1-3 substituents Y, which may be the same or different and are selected from C₁₋₃-alkyl or alkoxy, hydroxy, halogen, trifluoromethyl, trifluromethylthio, trifluromethoxy, nitro, amino, mono- or dialkyl (C₁₋₂)-amino, mono- or dialkyl (C₁₋₃)-alkyl sulfonyl, dimethylsufamido, C₁₋₃-alkoxycarbonyl, carboxyl, trifluromethylsulfonyl, cyano, carbamoyl and acetyl, or R₇ and/or R₈ represent naphtyl; R₉ represents hydrogen, hydroxyl, C₁₋₃-alkoxy, acetyloxy or propionyloxy;
- Aa represents one of the groups (i), (ii), (iii), (iv) or (v) 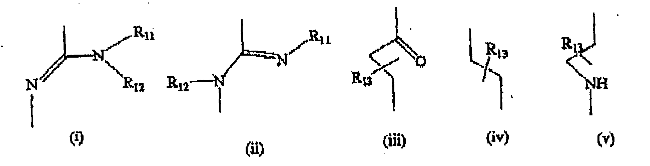 where
- R₁₁ and R₁₂ independently of each other represent hydrogen or C₁₋₈ branched or unbranched alkyl or C₃₋₈ cycloalkyl, or R₁₁ represents acetamido or dimethylamino or 2,2,2-trifluroethyl or phenyl or pyridyl with the proviso that R₁₂ represents hydrogen;
- R₁₃ represents hydrogen or C₁₋₃ unbranched alkyl;
- Bb represents sulfonyl or carbonyl;
- R₁₀ represents benzyl, phenyl, thienyl or pyridyl which may be substituted with 1, 2 or 3 substituents Y, which can be the same or different, or R₁₀ represents C₁₋₈ branched or unbranched alkyl or C₃₋₆ cycloalkyl, or R₁₀ represents naphtyl.

4. Use according to any of the preceding clams, of the cannabinoid receptor ligand of formula.

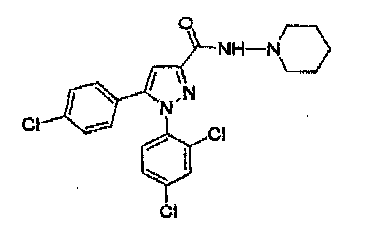

5. Use according to any of the preceding claims, of the cannabinoid receptor ligand of formula

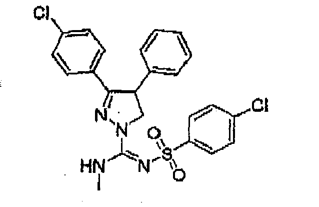

6. Use of a cannabinoid receptor ligand in the manufacture of a medicinal composition for modulating hair growth in a mammalian subject.

7. Use according to any of the preceding claims wherein the mammalian subject is a human.

8. A hair growth modulating composition comprising a cannabinoid receptor ligand as defined in any of claims 1 to 5.

9. A composition according to claim 8 also containing at least one other compound known for use as a hair growth modulator.

10. A composition according to claim 9, wherein the other compound known for use as a hair growth modulator is at least one of Dutaseride, Finasteride, Minoxidil, Fluorominoxidil, Fluridil, Viporstol, Trequinsin hydrochloride, Namindil and Procyanidin B-2.

11. A kit comprising a composition according to any of claims 8 to 10 and a pharmaceutically or cosmetically acceptable excipient.

12. A kit according to claim 11, further comprising a means to administer the cannabinoid receptor ligand.

13. A cosmetic method of modulating hair growth in mammalian subjects comprising administering a cannabinoid receptor ligand as defined in any of claims 1 to 5.

14. A cosmetic method of stimulating hair growth in mammalian subjects comprising administering a cannabinoid receptor ligand as defined in any of claims 1 to 5.

15. For modulating hair growth in mammalian subjects, a cannabinoid receptor ligand as defined in any of claims 1 to 5.

16. The compound of the formula
